(19)

**Europäisches Patentamt**
**European Patent Office**
**Office européen des brevets**

(11) **EP 2 364 681 B1**

(12) **EUROPÄISCHE PATENTSCHRIFT**

(45) Veröffentlichungstag und Bekanntmachung des Hinweises auf die Patenterteilung:
**27.07.2016 Patentblatt 2016/30**

(51) Int Cl.:
***A61F 9/008*** *(2006.01)*

(21) Anmeldenummer: **11162715.4**

(22) Anmeldetag: **04.10.2006**

(54) **Vorrichtung zur Materialbearbeitung mittels Laserstrahlung**

Device for materials processing using laser radiation

Dispositif de traitement de matériau par rayons laser

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **14.10.2005 DE 102005049281**

(43) Veröffentlichungstag der Anmeldung:
**14.09.2011 Patentblatt 2011/37**

(62) Dokumentnummer(n) der früheren Anmeldung(en) nach Art. 76 EPÜ:
**06792359.9 / 1 933 789**

(73) Patentinhaber: **Carl Zeiss Meditec AG**
**07745 Jena (DE)**

(72) Erfinder:
• **Bischoff, Mark**
**07749, Jena (DE)**
• **Mühlhoff, Dirk**
**07751, Jena (DE)**
• **Stobrawa, Gregor**
**07743, Jena (DE)**

(74) Vertreter: **Patentanwälte Geyer, Fehners & Partner mbB**
**Perhamerstrasse 31**
**80687 München (DE)**

(56) Entgegenhaltungen:
**WO-A1-2005/011546     DE-A1- 10 202 036**
**US-A1- 2003 212 387     US-A1- 2005 165 387**

EP 2 364 681 B1

**Beschreibung**

Die Erfindung bezieht sich auf eine Vorrichtung zur Materialbearbeitung mittels Laserstrahlung, mit einer Laserstrahlungsquelle, die gepulste Laserstrahlung zur Wechselwirkung mit dem Material abgibt, einer die gepulste Bearbeitungs-Laserstrahlung entlang einer optischen Achse in das Material auf ein Wechselwirkungszentrum fokussierenden Optik, einer die Lage des Wechselwirkungszentrums im Material verstellenden Scaneinrichtung, wobei jeder Bearbeitungs-Laserpuls in einer das ihm zugeordnete Wechselwirkungszentrum umgebenden Zone mit dem Material wechselwirkt, so dass in den Wechselwirkungszonen Material getrennt wird, und einer Steuereinrichtung, welche die Scaneinrichtung und die Laserstrahlquelle so ansteuert, dass im Material durch Aneinanderreihen von Wechselwirkungszonen eine Schnittfläche entsteht.

Bei Verfahren zur Materialbearbeitung mittels Laserstrahlung wird gepulste Laserstrahlung erzeugt und zur Wechselwirkung ins Material längs einer optischen Achse auf Wechselwirkungszentren fokussiert, und die Lage der Wechselwirkungszentren im Material verstellt, wobei jeder Bearbeitungs-Laserpuls in einer Zone um das ihm zugeordnete Wechselwirkungszentrum mit dem Material wechselwirkt und in den Wechselwirkungszonen Material trennt, wodurch eine Schnittfläche im Material durch Aneinanderreihen von Wechselwirkungszonen erzeugt wird.

Solche Vorrichtungen und Verfahren sind aus DE 10202036 A1, WO 2005/011546 A1, US 2005/165387 A1 und US 2003/212387 A1 bekannt.

Diese Vorrichtungen sowie entsprechende Verfahrenen zur Materialbearbeitung eignen sich besonders, um gekrümmte Schnittflächen innerhalb eines transparenten Materials auszubilden. Gekrümmte Schnittflächen innerhalb eines transparenten Materials werden beispielsweise bei laserchirurgischen Verfahren und dort insbesondere bei augenchirurgischen Eingriffen erzeugt. Dabei wird Behandlungs-Laserstrahlung in das Gewebe, d.h. unterhalb der Gewebeoberfläche auf ein Wechselwirkungszentrum fokussiert. In einer darum liegenden Wechselwirkungszone werden dadurch Materialschichten getrennt. Die Zone entspricht in der Regel dem Fokusspot. Üblicherweise wird die Laserpulsenergie so gewählt, dass in der Wechselwirkungszone ein optischer Durchbruch im Gewebe entsteht.

Im Gewebe laufen nach einen optischen Durchbruch zeitlich hintereinander mehrere Prozesse ab, die durch den Laserstrahlungspuls initiiert werden. Der optische Durchbruch erzeugt zuerst im Material eine Plasmablase. Diese Plasmablase wächst nach Entstehen durch sich ausdehnendes Gas. Anschließend wird das in der Plasmablase erzeugte Gas vom umliegenden Material aufgenommen und die Blase verschwindet wieder. Dieser Vorgang dauert allerdings sehr viel länger, als die Entstehung der Blase selbst. Wird ein Plasma an einer Materialgrenzfläche erzeugt, die durchaus auch innerhalb einer Materialstruktur liegen kann, so erfolgt ein Materialabtrag von der Grenzfläche. Man spricht dann von Photoablation. Bei einer Plasmablase, die vorher verbundene Materialschichten trennt, ist üblicherweise von Photodisruption die Rede. Der Einfachheit halber werden all solche Prozesse hier unter dem Begriff Wechselwirkung zusammengefasst, d.h. dieser Begriff schließt nicht nur den optischen Durchbruch sondern auch die anderweitigen materialtrennenden Wirkungen ein.

Für eine hohe Genauigkeit eines laserchirurgischen Verfahrens ist es unumgänglich, eine hohe Lokalisierung der Wirkung der Laserstrahlen zu gewährleisten und Kollateralschäden in benachbartem Gewebe möglichst zu vermeiden. Es ist deshalb im Stand der Technik üblich, die Laserstrahlung gepulst anzuwenden, so dass der zur Auslösung eines optischen Durchbruchs nötige Schwellwert für die Energiedichte nur in den einzelnen Pulsen überschritten wird. Die US 5.984.916 zeigt diesbezüglich deutlich, dass die räumliche Ausdehnung der Wechselwirkungszone nur dann wesentlich von der Pulsdauer abhängt, solange eine Pulslänge von 2 ps überschritten ist. Bei Werten von wenigen 100 fs ist die Wechselwirkungszonengröße nahezu pulsdauerunabhängig. Eine hohe Fokussierung des Laserstrahls in Kombination mit sehr kurzen Pulsen, d.h. unter 1 ps, erlaubt es damit, die Wechselwirkungszone punktgenau in einem Material einzusetzen.

Der Einsatz von solcher gepulster Laserstrahlung hat sich in der letzten Zeit besonders zur laserchirurgischen Fehlsichtigkeitskorrektur in der Ophthalmologie durchgesetzt. Fehlsichtigkeiten des Auges rühren oftmals daher, dass die Brechungseigenschaften von Hornhaut und Linse keine optimale Fokussierung auf der Netzhaut bewirken. Diese Art der Pulsung ist auch Gegenstand der hier geschilderten Erfindung.

Die erwähnte US 5.984.916 beschreibt ein Verfahren zur Schnitterzeugung mittels geeigneter Erzeugung optischer Durchbrüche, so dass im Endeffekt die Brechungseigenschaften der Hornhaut gezielt beeinflusst werden. Eine Vielzahl von optischen Durchbrüchen wird so aneinandergesetzt, dass die Schnittfläche innerhalb der Hornhaut des Auges ein linsenförmiges Teilvolumen isoliert. Das vom übrigen Hornhautgewebe getrennte linsenförmige Teilvolumen wird dann über einen seitlich öffnenden Schnitt aus der Hornhaut herausgenommen. Die Gestalt des Teilvolumens ist so gewählt, dass nach Entnahme die Form und damit die Brechungseigenschaften der Hornhaut so geändert sind, dass die erwünschte Fehlsichtigkeitskorrektur bewirkt ist. Die dabei geforderte Schnittfläche ist gekrümmt und umschreibt das Teilvolumen, was eine dreidimensionale Verstellung des Fokus nötig macht. Es wird deshalb eine zweidimensionale Ablenkung der Laserstrahlung mit gleichzeitiger Fokusverstellung in einer dritten Raumrichtung kombiniert. Dies wird hier unter dem Begriff "scannen", "verstellen" oder "ablenken" subsumiert.

Beim Aufbau der Schnittfläche durch Aneinanderreihen optischer Durchbrüche im Material verläuft die Erzeugung eines optischen Durchbruches um ein Vielfaches schneller, als es dauert, bis ein davon erzeugtes Plasma wieder im Gewebe absorbiert wird. Aus der Veröffentlichung A. Heisterkamp et al., Der Ophthalmologe, 2001, 98:623-628, ist es bekannt, dass nach Erzeugen eines optischen Durchbruches in der Augenhornhaut am Fokuspunkt, an dem der optische Durchbruch erzeugt wurde, eine Plasmablase entsteht, die mit Nachbarblasen zu Makroblasen zusammenwachsen kann. Die Veröffentlichung führt aus, dass das Zusammenschließen noch anwachsender Plasmablasen die Schnittqualität mindert. Es wird deshalb dort ein gattungsgemäßes Verfahren vorgeschlagen, bei dem einzelne Plasmablasen nicht direkt nebeneinander erzeugt werden. Stattdessen wird in einer spiralförmigen Bahn zwischen aufeinanderfolgend erzeugten optischen Durchbrüchen eine Lücke gelassen, die in einem zweiten Durchlauf durch die Spirale mit optischen Durchbrüchen und daraus resultierenden Plasmablasen gefüllt wird. Damit soll ein Zusammenschluss benachbarter Plasmablasen während des Anwachsens verhindert und die Schnittqualität verbessert werden.

Um eine gute Schnittqualität zu erreichen, verwendet der Stand der Technik also bestimmte Abfolgen, in denen die optischen Durchbrüche erzeugt werden. So soll ein Zusammenschließen anwachsender Plasmablasen verhindert werden. Da natürlich ein Schnitt angestrebt ist, bei dem möglichst wenig Brücken das Material bzw. Gewebe verbinden, müssen letztlich die erzeugten Plasmablasen auf jeden Fall zu einer Schnittfläche zusammenwachsen. Ansonsten blieben Materialverbindungen und der Schnitt wäre unvollständig.

Der Erfindung liegt deshalb die Aufgabe zugrunde, die Schnitte guter Qualität im Material zu erzeugen, ohne auf bestimmte Abfolgen der Laserpulseinbringung festgelegt zu sein.

Erfindungsgemäß wird die Aufgabe gelöst durch eine Vorrichtung gemäß Anspruch 1, bei der die Steuereinrichtung die Laserstrahlquelle und die Scaneinrichtung so ansteuert, dass die Schnittfläche zwei entlang der optischen Achse benachbarte Abschnitte aufweist und diese zumindest teilweise in einem zeitlichen Abstand $t \leq 5\,s$ mit Laserpulsen beleuchtet.

Die Erfindung geht von der Erkenntnis aus, dass sich Wechselwirkungszonen im Material gegenseitig beeinflussen. Die Wirkung eines Laserstrahlpulses hängt also davon ab, inwieweit in der Umgebung des Wechselwirkungszentrums bereits vorangegangene Lasereinwirkungen stattfanden. Daraus folgerten die Erfinder, dass die zur Erzeugung eines optischen Durchbruchs bzw. zur Bewirkung einer Materialtrennung erforderliche Pulsenergie vom Abstand zum nächstgelegenen Wechselwirkungszentrum abhängt. Die erfindungsgemäßen Varianten nutzen sämtlich diese Erkenntnis.

Eine optionale Minimierung des Abstandes von Wechselwirkungszentren, z. B. des Abstandes der Fokuslage benachbarter optischer Durchbrüche, ermöglicht eine Absenkung der Bearbeitungs-Pulsenergie. Der die Pulsenergie beschreibende Parameter ist die Fluence, also die Energie pro Fläche bzw. Energieflächendichte. Ein optionaler Abstand unter $10\,\mu m$ spricht also einen Aspekt der den Erfindern erstmals zuzuschreibenden Erkenntnis an.

Ein anderer Aspekt liegt darin, dass nun optional die Fluence der Bearbeitungs-Laserpulse deutlich gesenkt wird, man schreibt nun keine Obergrenze für den Abstand, sondern für die Fluence vor.

Die Erfindung stellt also Rahmenbedingungen für die Erzeugung eines Schnittes durch Einbringung gepulster Laserstrahlung auf, wobei die Rahmenbedingungen die Auswirkungen des unmittelbar benachbarten eingebrachten Pulses berücksichtigen. Für die Pulslänge wird hier die Lehre der US 5.984.916 angewendet, also Pulse unter 1 ps, vorzugsweise wenige 100 fs, z. B. 300 - 500 fs. Soweit die Erfindung eine Obergrenze des Abstandes definiert, ist der Abstand zum örtlich nächst gelegenen Wechselwirkungszentrum gemeint. Da eine Schnittfläche in der Regel durch eine Vielzahl von aneinandergereihten Wechselwirkungszentren erzeugt wird, kann unter dem Abstand zur Vereinfachung auch der Mittelwert des Abstandes von Laserfoki für die einzelnen Laserpulse im Material verstanden werden. Wenn das entlang einer Schnittfläche im wesentlichen zweidimensionale Gitter aus Wechselwirkungszentren nicht symmetrisch ist, kann mit dem Abstand auch der charakteristische mittlere Abstand gemeint sein. Im Stand der Technik ist es bekannt, eine gepulste Laserstrahlungsquelle zu verwenden und einen Teil der davon abgegebenen Laserstrahlungsquelle so zu verändern, dass sie keinen Bearbeitungseffekt im Material auslösen. Nur ein Teil der Laserstrahlungspulse dient dann zur Bearbeitung. Soweit für die hier vorliegende Beschreibung des Begriffs "Laserstrahlungspuls", "Laserpuls" oder "Puls" verwendet wird, ist immer ein Bearbeitungs-Laserpuls gemeint, also ein Laserstrahlungspuls der zur Wechselwirkung mit dem Material vorgesehen bzw. ausgebildet bzw. geeignet ist.

Der apparative Aufwand sinkt durch die Erfindung, weil die Pulsspitzenleistung abnimmt. Durch den reduzierten Abstand der Wechselwirkungszentren steigt die Pulsfolgefrequenz, wenn die Bearbeitungsdauer konstant gehalten wird. Weiter werden im Falle optischer Durchbrüche kleinere Plasmablasen erzeugt, wodurch der Schnitt feiner wird. Im Stand der Technik wurde hingegen immer mit vergleichsweise größeren Abständen der Wechselwirkungszentren gearbeitet und die Fluence der Pulse entsprechend hoch gewählt, um gesichert optische Durchbrüche und entsprechend an die Abstände angepasste, große Plasmablasen zu erhalten.

Eine niedrigere Fluence reduziert zugleich auch die Personengefährdung bei der Materialbearbeitung. Bei augenchirurgischen Verfahren ist dies von wesentlicher Bedeutung. Als besonderer Vorteil stellt sich dabei heraus, dass nunmehr mit Lasern der Gefährdungsklasse 1M gearbeitet werden kann, wohingegen im Stand der Technik die Laserklasse 3 nötig war. Diese Klasse verlangte, dass Bedienpersonal, beispielsweise ein Arzt oder eine Krankenschwester, Schutzbrillen tragen, was von Patienten naturgemäß als beunruhigend empfunden wird. Solche Schutzmaßnahmen sind mit den erfindungsgemäß nun möglichen Laser der Klasse 1 M nicht mehr nötig.

Es ist deshalb als Weiterbildung vorgesehen eine Vorrichtung zur Materialbearbeitung mittels Laserstrahlung, mit einer abgebenden Laserstrahlquelle, die gepulste Laserstrahlung zur Wechselwirkung mit dem Material abgibt, einer die gepulste Laserstrahlung in das Material auf ein Wechselwirkungszentrum fokussierenden Optik, einer die Lage des Wechselwirkungszentrums im Material verstellenden Scaneinrichtung, wobei jeder Bearbeitungs-Laserpuls in einer das ihm zugeordnete Wechselwirkungszentrum umgebenden Zone mit dem Material wechselwirkt, so dass in den Wechselwirkungszonen Material getrennt wird, und mit einer Steuereinrichtung, welche die Scaneinrichtung und die Laserstrahlquelle so ansteuert, dass im Material durch Aneinanderreihen von Wechselwirkungszonen eine Schnittfläche entsteht, wobei ein Laser einer Gefährdungsklasse unter 3, vorzugsweise ein Laser der Gefährdungsklasse 1M, zum Einsatz kommt. Die Angabe der Gefährdungsklasse bezieht sich auf die internationale Norm IEC 60825-1 in der am 13. Oktober 2005 geltenden Fassung. Analog ist (eigenständig oder als Weiterbildung) vorgesehen eine Vorrichtung zur Materialbearbeitung mittels Laserstrahlung, mit einer Laserstrahlquelle, die gepulste Laserstrahlung zur Wechselwirkung mit dem Material abgibt, einer die gepulste Laserstrahlung entlang einer optischen Achse in das Material auf ein Wechselwirkungszentrum fokussierenden Optik, einer die Lage des Wechselwirkungszentrums im Material verstellenden Scaneinrichtung wobei jeder Laserpuls in einer das zugeordnete Wechselwirkungszentren umgebenden Zone mit dem Material wechselwirkt und in den Wechselwirkungszonen Material getrennt wird, und mit einer Steuereinrichtung, welche die Scaneinrichtung und die Laserstrahlquelle so ansteuert, dass im Material durch Aneinanderreihen von Wechselwirkungszonen eine Schnittfläche entsteht, wobei ein Laser einer Gefährdungsklasse unter 3, vorzugsweise ein Laser der Gefährdungsklasse 1M, verwendet wird. Dies bietet sich auch als Weiterbildung für jede der genannten Vorrichtungen bzw. für jedes der genannten Verfahren an. Soweit nicht explizit ausgeführt, gilt dies nachfolgend für jede geschilderte vorteilhafte Ausgestaltung, Weiterbildung oder Ausführung.

Von den Erfindern durchgeführte Untersuchungen zeigten, dass ein optischer Durchbruch erst oberhalb eines bestimmten Schwellwertes M einsetzt, der vom Abstand a benachbarter Wechselwirkungszentren gemäß der Gleichung $M = 3{,}3 \text{ J/cm}^2 - (2{,}4 \text{ J/cm}^2) / (1 + (a/r)^2)$ abhängt. Erst bei einer Puls-Fluence über dem Schwellwert M ist ein optischer Durchbruch durch jeden einzelnen Laserpuls gesichert. Der in der Gleichung auftretende Parameter r stellt dabei eine experimentell erkannte mittlere Reichweite der Beeinflussung benachbarter Wechselwirkungszonen dar. Applikationsabhängig können hier Schwankungen vorliegen, so dass eine Variation des Wertes zwischen 3 und 10 $\mu$m möglich ist, vorzugsweise gilt $r = 5$ $\mu$m.

Die optional erwähnte Obergrenze der Puls-Fluence wird in einer Weiterbildung der Erfindung auch auf die genannte Abhängigkeit des Schwellwertes vom Abstand benachbarter Wechselwirkungszentren bezogen werden. Es ist deshalb eine Weiterbildung bevorzugt, bei der die Fluence um eine Überschussenergie von maximal 3 J/cm$^2$ über dem Schwellwert M liegt. Der dadurch definierte Bereich liefert eine besonders gute Schnittqualität, gleichzeitig ist die Einleitung eines optischen Durchbruches sichergestellt. Würde man die Überschussenergie weiter vergrößern, würden unnötig große Plasmablasen erzeugt und die Schnittqualität verschlechterte sich.

Es muss zur Schnitterzeugung nun aber nicht mehr zwingend mit optischen Durchbrüchen gearbeitet werden. Auch mit Energie der gepulsten Laserstrahlung, die unter einem Schwellwert zur Einleitung eines optischen Durchbruches liegt, kaum nach Erkenntnis der Erfinder eine Materialtrennung und damit eine Schnittflächenbildung erfolgen, wenn sich Wechselwirkungszonen überlappen. Es ist deshalb eine Weiterbildung vorgesehen, bei der der örtliche Abstand a der Wechselwirkungszentren zweier aufeinanderfolgender Pulse kleiner ist als die Fokusgröße d, so dass aufeinanderfolgend mit Laserstrahlung beaufschlagte Volumina des Materials, also Wechselwirkungszonen, sich gegenseitig überlappen. Durch diese Ausgestaltung erfolgt Materialtrennung ohne Plasmablasenbildung, was zu einem besonders glatten Schnitt führt.

Vorteilhafterweise kann dann die Fluence des Laserpulses auch unter den bereits erläuterten Schwellwert abgesenkt werden, da durch Überlagerung von Wechselwirkungszonen insgesamt dennoch ein gewebetrennender Effekt erreicht wird. Der einzelne Laserpuls erzeugt dann nicht mehr sicher einen optischen Durchbruch; erst die Überlagerung von Wechselwirkungszonen bewirkt die Gewebetrennung. Dies erlaubt Puls-Energien, die um Größenordnungen unter den bislang üblichen liegen, zugleich wird die Schnittqualität nochmals gesteigert, da sich zeitlich aufeinanderfolgend erzeugte Wechselwirkungszonen überdecken. Der Abstand der Wechselwirkungszentren reicht also von Null bis zum Durchmesser des Fokus, welcher beispielsweise zwischen 1 und 5 $\mu$m liegt, wenn man den $1/e^2$-Durchmesser (e = Eulersche Zahl) betrachtet.

Die optionale Schnitterzeugung bewirkt einen sehr feinen Schnitt, da aufgrund des reduzierten Abstandes bzw. der reduzierten Pulsenergie mit entsprechend kleinen oder sogar ganz ohne Plasmablasen gearbeitet wird bzw. gearbeitet werden kann. Eine feine Schnittfläche kann aber auch nachteilig sein, beispielsweise wenn ein Bediener die Schnittfläche zumindest teilweise optisch erkennen muss. Dies ist beispielsweise bei der Laserchirurgie nach dem fs-LASIK-Verfahren der Fall. Das dort durch die Einwirkung der Laserstrahlung isolierte Teilvolumen, das durch einen seitlichen Schnitt aus dem Gewebe entnommen werden soll, wird üblicherweise durch einen Operator zuerst mittels eines Spatels von etwaigen Restbrücken zum umgebenden Material befreit. Dazu schiebt der Operator den Spatel in die durch den seitlich öffnenden Schnitt erzeugte Tasche und fährt das Teilvolumen mit dem Spatel ab. Bei einer sehr feinen, also glatten Schnittfläche kann es vorkommen, dass der Operateur den Verlauf der Schnittfläche im Material nicht mehr von außen sehen kann. Er weiß deshalb nicht, wo der Rand des Teilvolumens liegt, und kann den Spatel nicht sicher führen. Zur Lösung dieser Problematik ist ein Verfahren der eingangs genannten Art vorgesehen, bei dem die Schnittfläche in mindestens zwei Teilflächen aufgeteilt wird und eine Teilfläche mit Betriebsparametern erzeugt wird, die eine gröbere also rauhere Schnittfläche erzeugen. Bei einer gattungsgemäßen Vorrichtung führt die Steuereinrichtung die entsprechende Ansteuerung von Laserquelle und Scaneinrichtung aus. Vorzugsweise wird man diese gröbere Schnittfläche an den Rand legen, der dadurch für den Benutzer einfach erkennbar ist und für die Qualität der Schnittfläche, z. B. bei der Augenchirurgie, keine Rolle spielt. Die beiden Teilflächen unterscheiden sich also hinsichtlich mindestens eines die Feinheit der Schnittfläche beeinflussenden Parameters. Ein möglicher Parameter ist beispielsweise die Fluence der verwendeten Laserpulse oder der örtliche Abstand der Wechselwirkungszentren.

Kombiniert man diesen Ansatz, der prinzipiell auf verschiedene Art und Weisen ausgeführt werden kann und nicht auf die hier geschilderte Erfindung beschränkt ist, mit einer der genannten Optionen, ist es zweckmäßig, dass die Steuereinrichtung die Laserstrahlquelle und die Scaneinrichtung so ansteuert, dass die Schnittfläche aus mindestens einer ersten und einer zweiten Teilschnittfläche aufgebaut ist, wobei die erste Teilschnittfläche mit einer Ansteuerung der Laserstrahlquelle und der Scaneinrichtung nach einem der vorgenannten erfindungsgemäßen Konzepte und die zweite Teilschnittfläche mit einer Ansteuerung der Laserstrahlquelle erfolgt, die eine Puls-Fluence über 3 J/cm$^2$ vorzugsweise über 5 J/cm$^2$ bewirkt. Dabei kann natürlich auch a > 10 $\mu$m eingestellt werden, da die Plasmablasen dann groß sind. Letztere Teilfläche weist dann automatisch die erwünschte gröbere Struktur auf und erleichtert dem Bediener bzw. Operateur das Erkennen der Schnittfläche. Das analoge Verfahren sieht entsprechend vor, dass die zweite Teilschnittfläche mit einem der erfindungsgemäßen Verfahren mit einer Puls-Fluence über 3 J/cm$^2$, vorzugsweise über 5 J/cm$^2$, erzeugt wird.

Zweckmäßigerweise wird man die gröbere Teilfläche so wählen, dass sie die feinere Teilfläche umgibt, so dass der Operateur den Rand der Schnittfläche gut erkennen kann und keine Nachteile für die optische Abbildung (im Falle der Augenchirurgie) am behandelten Auge entstehen.

Die der Erfindung zugrunde liegende Erkenntnis zeigt weiter, dass mit einem abnehmenden Abstand der Wechselwirkungszentren der Schwellwert, der zur gesicherten Erreichung eines optischen Durchbruchs nötig ist, abfällt.

Die von den Erfindern durchgeführte Analyse zeigte weiter, dass die Gestalt der erzeugten Plasmablasen, welche als Ergebnis der Wechselwirkung der Laserpulse mit dem Material bzw. Gewebe entstehen, einer zeitlichen Änderung unterliegen kann, wie es auch die Veröffentlichung von Heisterkamp et al. andeutete. Während diese Veröffentlichung sich aber darauf konzentriert, zu verhindern, dass ein Wechselwirkungszentrum nahe einer gerade anwachsenden Plasmablase liegt, hebt die Erfindung nun darauf ab, dass sich die Deformation einer Makroblase nicht auf die Schnittqualität auswirkt. Würde in ein deformiertes Material oder Gewebe an eine bestimmte Position ein weiterer optischer Durchbruch gesetzt, so verschöbe sich die Lage des Wechselwirkungszentrums im Material oder Gewebe, sobald die Deformation durch Relaxation zurückgeht. Es ist deshalb erfindungsgemäß vorgesehen, die Zeit zwischen der Applikation der Laserenergie in zwei sich potentiell beeinflussenden Gebieten des Materials bzw. Gewebes so gering zu halten, dass sie kleiner ist, als eine charakteristische Zeit für die Bildung von Makroblasen. Diese liegt bei etwa 5 s. Dieses Vorgehen ist natürlich nur dann erforderlich, wenn zwei entlang der optischen Achse benachbarte Abschnitte der Schnittfläche vorhanden sind, da nur dann eine Deformation, die durch Erzeugung eines Schnittflächen-Abschnittes erzeugt wurde, sich auf die Ausbildung des anderen, entlang der optischen Achse benachbarten Schnittflächen-Abschnittes auswirken kann.

Besonders bedeutsam ist dieses Vorgehen bei der Erzeugung eines Teilvolumens beim fs-LASIK-Verfahren. Dieses auch als Lentikel bezeichnete Teilvolumen wird durch einen posterioren und einen anterioren Abschnitt der Schnittfläche erzeugt, so dass die Schnittfläche insgesamt das Lentikel umschreibt. Posterioren und anterioren Abschnitt zusammen innerhalb der charakteristischen Zeit zur Bildung der Makroblasen zu erzeugen, kann jedoch relativ hohe Anforderungen an die Ablenkgeschwindigkeit der Scaneinrichtung mit sich bringen oder erzwingt bestimmte Scanbahnen. Die kann vorzugsweise vermieden werden, wenn man den posterioren und den anterioren Abschnitt in Teilflächen aufteilt und die Bearbeitungsabfolge dieser Teilflächen geschickt wählt.

In einer Ausgestaltung werden die beiden Flächen in ringförmige Teilflächen unterteilt. Da bei einem Lentikel die zentrale Teilfläche die optische Qualität sehr viel stärker beeinflusst die Randgebiete, wird zuerst der Schnitt entsprechend der zentralen Teilfläche des posterioren Abschnittes und dann der des anterioren Abschnittes erzeugt, so dass die Teilflächen zeitlich unmittelbar benachbart gebildet werden. Dann wird die ringförmige Teilfläche des posterioren Abschnittes und anschließend des anterioren Abschnittes geschnitten. Dieses Prinzip kann auch mit beliebig vielen Teilflächen ausgeführt werden. Die praktischen Grenzen ergeben sich dadurch, dass zum Wechsel zwischen anteriorem und posteriorem Abschnitt immer der Laserfokus entlang der optischen Achse verändert werden muss, was aus technischen Gründen die meiste Zeit bei der Ablenkung in Anspruch nimmt.

Bei diesem Vorgehen ist es wichtig zu beachten, dass der Durchmesser einer jeden ring- bzw. kreisförmigen posterioren Teilfläche etwas größer sein muss, als der Durchmesser der anschließend erzeugten jeweiligen anterioren Teilfläche. Das stellt sicher, dass der als nächstes zu erzeugende, posteriore Teilschnitt nicht nur anterior liegende, als Streuzentren wirkende Disruptionsblasen unmöglich gemacht wird. Das Mindestmaß, um welches der posteriore Teilschnitt größer sein muss, als der zugeordnete anteriore Teilschnitt, ergibt sich aus der numerischen Apertur der fokussierenden Optik.

Eine weitere Möglichkeit, den zeitlichen Abstand unter die charakteristische Zeit zu drücken, besteht darin, den posterioren Abschnitt mit einer von außen nach innen verlaufenden Spirale der Wechselwirkungszentren zu erzeugen, und den anterioren Abschnitt mit einer von innen nach außen verlaufenden Spirale. Damit ist sichergestellt, dass entlang der optischen Achse benachbarte Abschnitte zumindest im zentralen Bereich innerhalb des zeitlichen Abstandes von 5 s gebildet werden. Dieses Verfahren lässt sich natürlich auf die bereits erwähnten Teilflächenaufteilungen anwenden.

Es ist deshalb bevorzugt, dass die Steuereinrichtung die Laserstrahlquelle sowie die Scaneinrichtung so ansteuert, dass die der optischen Achse benachbarten Abschnitte zumindest teilweise unmittelbar zeitlich aufeinanderfolgend durch das Aneinanderreihen der Wechselwirkungszentren beleuchtet.

Die Erfindung wird nachfolgend unter Bezugnahme auf die Zeichnungen beispielhalber noch näher erläutert. In den Zeichnungen zeigt:

| | |
|---|---|
| Fig. 1 | ein laserchirurgisches Instrument zur Augenbehandlung, |
| Fig. 2 | eine Schemazeichnung der Wirkung der Laserstrahlung auf die Augenhornhaut beim Instrument der Fig. 1, |
| Fig.3 | eine Schemadarstellung zur Veranschaulichung der Erzeugung und Isolierung eines Teilvolumens mit dem Instrument der Fig. 1, |
| Fig. 4 | eine Ablenkvorrichtung des Instruments der Fig. 1, |
| Fig. 5 | ein Blockdiagramm des Aufbaus des Instruments der Fig. 1, |
| Fig. 6 | einen Zusammenhang zwischen Abstand der Zentren der optischen Durchbrüche, die vom Instrument der Fig. 1 erzeugt werden, und der Pulsenergie, wobei mögliche Betriebsbereiche für das Instrument der Fig. 1 eingezeichnet sind, |
| Fig. 7 | eine Darstellung ähnlich der Fig. 6, |
| Fig. 8 | eine schematische Draufsicht auf die Augenhornhaut zur Verdeutlichung der Lage der erzeugten Plasmablasen bzw. der dadurch bewirkten Schnittfläche, |
| Fig. 9 | eine Schnittdarstellung durch die Darstellung der Fig. 8 gemäß der Linie A1-A1, |
| Fig. 10 | eine Schemadarstellung zur Anordnung mehrerer Wechselwirkungszonen bei der Erzeugung der Schnittfläche mit einem Instrument gemäß Fig. 1 und |
| Fig. 11 und 12 | Darstellungen ähnlich der Fig. 10 für abgewandelte Betriebsmodi. |

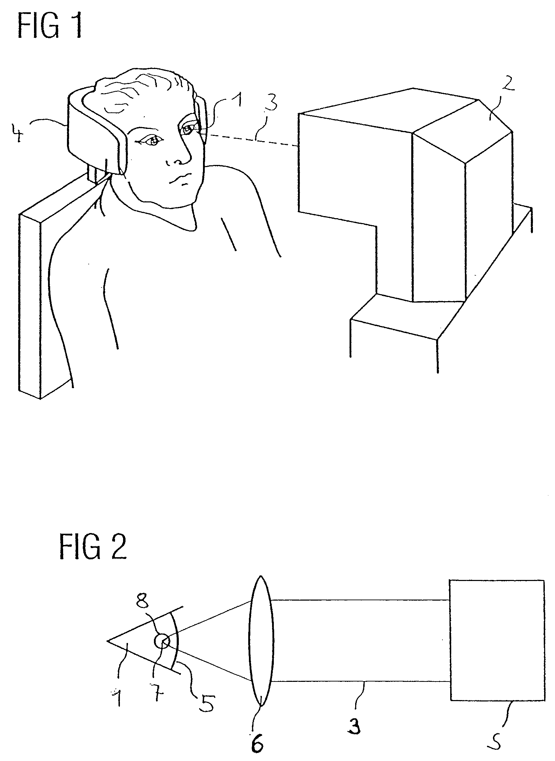

In Fig. 1 ist ein laserchirurgisches Instrument zur Behandlung eines Auges 1 eines Patienten gezeigt, wobei das laserchirurgische Instrument 2 zur Ausführung einer refraktiven Korrektur dient. Das Instrument 2 gibt dazu einen Behandlungs-Laserstrahl 3 auf das Auge des Patienten 1 ab, dessen Kopf in einem Kopfhalter 4 fixiert ist. Das laserchirurgische Instrument 2 ist in der Lage, einen gepulsten Laserstrahl 3 zu erzeugen, so dass das in US 5.984.916 beschriebene Verfahren ausgeführt werden kann. Der Behandlungs-Laserstrahl 3 besteht z. B. aus fs-Laserpulsen mit einer Pulswiederholfrequenz zwischen 10 und 500 kHz. Die Baugruppen des Instrumentes 2 werden im Ausführungsbeispiel von einer integrierten Steuereinheit gesteuert.

Das laserchirurgische Instrument 2 weist, wie in Fig. 2 schematisch dargestellt ist, eine Strahlquelle S auf, deren Strahlung in die Hornhaut 5 des Auges 1 fokussiert wird. Mittels des laserchirurgischen Instrumentes 2 wird eine Fehlsichtigkeit des Auges 1 des Patienten dadurch behoben, dass aus der Hornhaut 5 Material so entfernt wird, dass sich die Brechungseigenschaften der Hornhaut um ein gewünschtes Maß ändern. Das Material wird dabei dem Stroma der Hornhaut entnommen, das unterhalb von Epithel und Bowmanscher Membran und oberhalb von Decemetscher Membran und Endothel liegt.

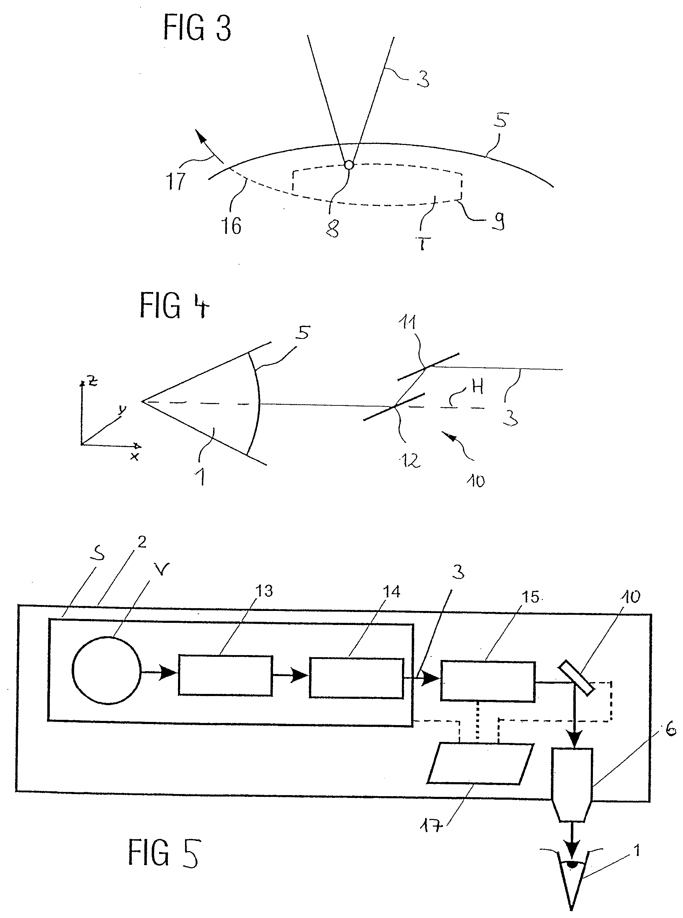

Die Materialentfernung erfolgt, indem durch Fokussierung des hochenergetischen gepulsten Laserstrahls 3 mittels eines verstellbaren Teleskopes 6 in einem in der Hornhaut 5 liegenden Fokus 7 in der Hornhaut Gewebeschichten getrennt werden. Jeder Puls der gepulsten Laserstrahlung 3 erzeugt dabei einen optischen Durchbruch im Gewebe, welcher wiederum eine Plasmablase 8 initiiert. Dadurch umfasst die Gewebeschichttrennung ein größeres Gebiet, als der Fokus 7 der Laserstrahlung 3, obwohl die Bedingungen zur Erzielung des Durchbruches nur im Fokus 7 erreicht werden. Durch geeignete Ablenkung des Laserstrahls 3 werden nun während der Behandlung viele Plasmablasen 8 erzeugt. Dies ist schematisch in Fig. 3 dargestellt. Die Plasmablasen bilden dann eine Schnittfläche 9, die ein Teilvolumen T des Stromas, nämlich das zu entfernende Material der Hornhaut 5 umschreiben. Durch Aneinanderreihung der Plasmablasen 8 in Folge stetiger Verschiebung des Fokus 7 des gepulsten fokussierten Laserstrahls 3 wird die Schnittfläche 9 gebildet.

Das laserchirurgische Instrument 2 wirkt durch die Laserstrahlung 3 wie ein chirurgisches Messer, das, ohne die Oberfläche der Hornhaut 5 zu verletzen, direkt Materialschichten im Inneren der Hornhaut 5 trennt. Führt man einen Schnitt 16 durch weitere Erzeugung von Plasmablasen 8 bis an die Oberfläche der Hornhaut, kann ein durch die Schnittfläche 9 isoliertes Material der Hornhaut 5 seitlich in Richtung des Pfeiles 17 herausgezogen und somit entfernt werden.

Die Fokusverschiebung erfolgt dabei zum einen in einer Ausführungsform mittels der in Fig. 4 schematisch dargestellten Ablenkeinheit 10, die den auf einer optischen Achse H auf das Auge 1 einfallenden Laserstrahl 3 um zwei senkrecht zueinander liegenden Achsen ablenkt. Die Ablenkeinheit 10 verwendet dafür einen Zeilenspiegel 11 sowie einen Bildspiegel 12, was zu zwei hintereinander liegenden räumlichen Ablenkachsen führt. Der Kreuzungspunkt der optischen Achse H mit der Ablenkachse ist dann der jeweilige Ablenkpunkt. Zur Fokusverschiebung wird zum anderen das Teleskop 6 geeignet verstellt. Dadurch kann der Fokus 7 in dem in Fig. 4 schematisch dargestelltem x/y/z-Koordinatensystem entlang dreier orthogonaler Achsen verstellt werden. Die Ablenkeinheit 10 verstellt den Fokus in der x/y-Ebene, wobei der Zeilenspiegel den Fokus in der x-Richtung und der Bildspiegel in der y-Richtung zu verstellen erlaubt. Das Teleskop 6 wirkt dagegen auf die z-Koordinate des Fokus 7. Somit ist insgesamt eine dreidimensionale Ablenkung des Fokus 7 erreicht.

Aufgrund der Cornea-Krümmung, die zwischen 7 und 10 mm beträgt, muss das Teilvolumen T auch entsprechend gekrümmt sein. Die Cornea-Krümmung erfordert somit eine der Schnittebene. Diese wird durch geeignete Ansteuerung der Ablenkeinheit 10 und des Teleskopes 6 bewirkt.

Die Fig. 5 zeigt ein vereinfachtes Blockschaltbild des laserchirurgischen Instrumentes 2 für die refraktive Chirurgie am menschlichen Auge 1. Dargestellt sind nur die wichtigsten Baugruppen: ein als Strahlquelle S dienender fs-Laser, welcher aus einem fs-Oszillator V, sowie einer oder mehreren Verstärkerstufen 13 besteht und dem hier noch ein Kompressor bzw. Pre-Kompressor 14 nachgeordnet ist; ein Laserpulsmodulator 15, der mit der Laserstrahlung aus dem Laser S beaufschlagt wird; die Ablenkeinheit 10, hier als Scanner realisiert; ein das Teleskop 6 verwirklichendes Objektiv zur Fokussierung in das zu bearbeitende Gewebe, und die Steuereinheit 17.

Der Laser S erzeugt Laserpulse mit einer Dauer im fs-Bereich. Die Laserpulse gelangen zunächst in den Laserpulsmodulator 15, der (auf noch zu beschreibende Art) die Laserpulse gemäß einem Steuersignal der Steuereinheit 17 beeinflusst. Anschließend gelangen zumindest die Behandlungs-Laserpulse zum Scanner 10 und durch das Objektiv 6 in das Patientenauge 1. Sie werden dort fokussiert und erzeugen im Fokus 7 optische Durchbrüche. Der Modulator stellt die Energie der Laserpulse, d. h. die Fluence der einzelnen Laserpulse ein. Als Modulator kann ein AOM oder auch ein elektrooptischer Modulator (EOM), eine Pockelszelle, ein Flüssigkristallelement (LC-Element), ein faseroptisches Schaltelement oder ein variables Abschwächelement, beispielsweise Graufilter, verwendet werden.

Das laserchirurgische Instrument 1 kann nun in verschiedenen Betriebsarten arbeiten, die jeweils für sich eigenständig oder in Kombination realisiert sein können und die die Energie bzw. Fluence F jedes Laserpulses bzw. den örtlichen Abstand, mit dem die Laserpulse zur Erzeugung der Schnittfläche 9 aneinandergereiht werden, betreffen.

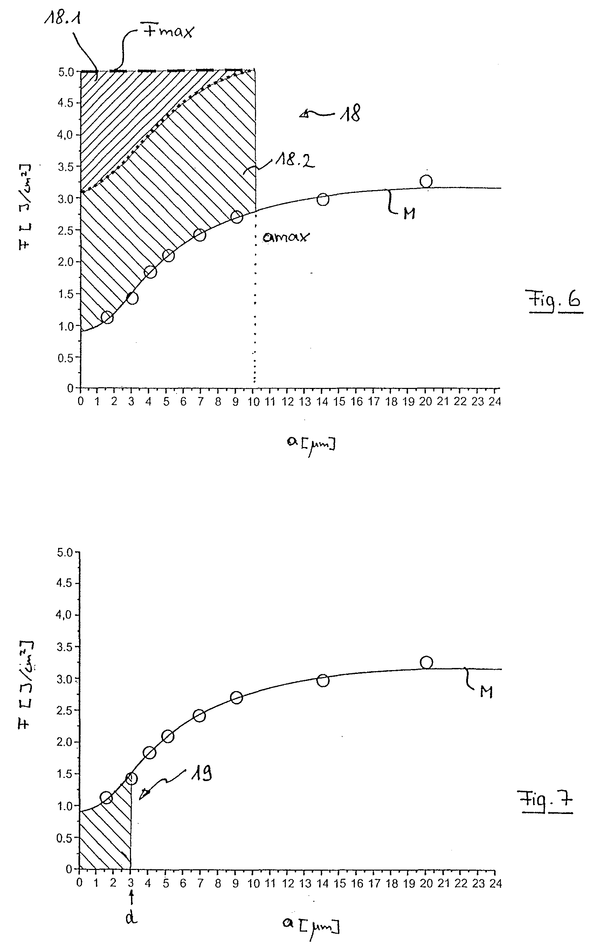

In Fig. 6 ist ein Schwellwert M als Kurve aufgetragen, der als Zusammenhang zwischen einem Abstand a, in dem die Wechselwirkungszentren der einzelnen Laserpulse in der Augenhornhaut 5 aneinandergereiht werden, und der Fluence F jedes Laserpulses wiedergibt. Nur bei einer Fluence über dem Schwellwert entsteht ein optischer Durchbruch mit anschließender Plasmablase.

Die an der Kurve gezeichneten kreisförmigen Eintragungen stammen aus experimentellen Vermessungen und stellen Messpunkte dar. Die Vermessungen erfolgte mit einer Pulslänge von 300 fs und einem Spotdurchmesser des Fokus 7 von 3 $\mu$m.

Das Instrument 1 kann in einem Betriebsgebiet 18 gemäß Fig. 6 betrieben werden, das durch verschiedene Randbedingungen definiert werden kann. Die unterschiedlichen Definitionen entsprechen unterschiedlichen Varianten der Erfindung. Alle Varianten nutzen den Verlauf des Schwellwertes M für die Fluence F als Funktion des Abstandes a aus. Diese Abhängigkeit ist näherungsweise durch folgende Formel gegeben: $M = 3,3\ J/cm^2 - (2,4\ J/cm^2) / (1 + (a/r)^2)$, wobei r ein Parameter ist, der die mittlere Reichweite der Beeinflussung wiedergibt und zwischen 3 und 10 $\mu$m, vorzugsweise 5 $\mu$m liegt.

In einer ersten Variante arbeitet das Instrument 1 mit einem Abstand a der Laserfoki 7, d. h. der Wechselwirkungszentren, der unter einem Maximalwert $a_{max}$ = 10 $\mu$m liegt. Ab diesem Wert fällt die Kurve für den Schwellwert M zu niedrigeren Abständen a hin deutlich ab, so dass es möglich ist, mit einer deutlich reduzierten Fluence F zu arbeiten.

In einer zweiten Variante wird mit einer Obergrenze $F_{max}$ für die Fluence F gearbeitet. Der Wert hierfür ist 5 $J/cm^2$.

In einer Kombination der ersten und zweiten Variante gilt sowohl $a \leq a_{max}$ als auch $F \leq F_{max}$. Die Abstände der Wechselwirkungszentren sowie die Fluence der Laserpulse befinden sich also innerhalb des aus noch zu erläuternden Teilflächen 18.1 und 18.2 aufgebauten Gebietes. Da das laserchirurgische Instrument 1 in beiden Varianten für sich wie auch in der Kombination dieser beiden Varianten jeweils optische Durchbrüche im Material, beispielsweise der Hornhaut 5, erzeugt, liegt die Fluence F natürlich immer über dem Schwellwert M, da erst oberhalb dieses Schwellwertes jeder Laserpuls gesichert einen optischen Durchbruch 8 erzeugt.

Eine dritte Variante wandelt die zweite Variante nun dahingehend ab, dass die Fluence F jedes Laserpulses den Schwellwert M nur maximal um eine Überschussenergie überschreitet, die zwischen 3 und 3,5 $J/cm^2$ liegt. Die

Fluence F wird dann unter der gepunkteten Linie der Fig. 6 gehalten, die die Bereiche 18.1 und 18.2 voneinander trennt. Natürlich kann auch die dritte Variante mit der ersten Variante kombiniert werden, wodurch Fluence F und Abstand a im schraffierten Gebiet 18.2 liegen.

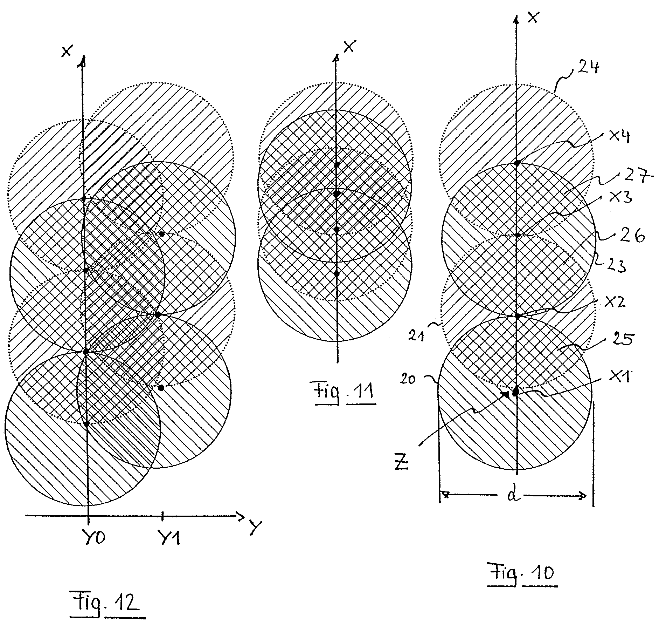

In einer anderen Ausgestaltung arbeitet das laserchirurgische Instrument 1 mit Laserpulsen, die nicht jeder gesichert einen optischen Durchbruch 8 erzeugen. Um dennoch eine Materialtrennung zu erreichen, sind die Wechselwirkungszentren in einem Abstand a aneinander angereiht, der geringer ist, als der Durchmesser d des Laserfokus, also geringer als die Größe der Wechselwirkungszonen. Diese Arbeitsweise ist in den Fig. 10 - 12 näher gezeigt.

Fig. 10 zeigt in einem eindimensionalen Beispiel die Anordnung der Wechselwirkungszentren Z, die der Lage des (theoretischen) Fokuspunkts entsprechen. Jede Wechselwirkung wird durch einen Laserpuls erzeugt, wobei der Fokus 7 z. B. beugungsbegrenzt ist und den Durchmesser d hat, beispielsweise 3 μm, wie es in Fig. 7 angenommen ist. Die Wechselwirkungszentren, d. h. das Zentrum der fokussierten Laserstrahlung wird nun so verschoben, dass benachbart abgedeckte Wechselwirkungszonen 20, 21, 23 und 24 jeweils mit ihren unmittelbaren Nachbarn überlappen. Es gibt somit Überdeckungsbereiche 25, 26, 27, die jeweils von zwei Wechselwirkungszonen abgedeckt sind. Die in eine Wechselwirkungszone eingebrachte Energie liegt unter dem Schwellwert M, so dass jede der Wechselwirkungszonen 20 - 24 für sich nicht gesichert einen optischen Durchbruch bewirkt. Aufgrund der Überdeckung wird aber dennoch eine materialtrennende Wirkung erreicht. Wesentlich für diese Arbeitsweise ist also, dass der Abstand zwischen den Koordinaten der Wechselwirkungszentren geringer ist, als die Ausdehnung d der Wechselwirkungszonen. In Fig. 10 ist deutlich zu sehen, dass der Abstand zwischen den einzelnen Koordinaten X1, X2, X3 und X4 etwa dem halben Durchmesser d der Wechselwirkungszonen 20 - 24 entspricht, wodurch eine einfache Überdeckung gegeben ist.

Fig. 11 zeigt eine engere Staffelung der Wechselwirkungszonen, so dass im Endeffekt eine vierfache Überdeckung der Wechselwirkungszonen gegeben ist. Dies erlaubt eine weitere Absenkung der Fluence F.

Fig. 12 veranschaulicht, dass die Darstellung der Figuren 10 und 11 lediglich der Einfachheit halber nur eindimensional, d. h. nur unter Berücksichtigung der x-Koordinate dargestellt sind. Eine Verschiebung der sich gegenseitig in x-Richtung überlagernden Wechselwirkungszonen in y-Richtung erreicht weitere Überdeckungen, so dass trotz der an und für sich nur einfachen Überdeckung in x-Richtung je nach Beabstandung in y-Richtung eine drei- oder fünffache Überdeckung von Wechselwirkungszonen erreicht wird. Die Wahl der Abstände in x-Richtung bzw. y-Richtung erlaubt hier beliebige Überdeckungsfaktoren (2, 3, 4, 5, 6, 7, ...).

Im Ergebnis arbeitet das Instrument 1 im Betriebsbereich 19, der dadurch gekennzeichnet ist, dass der Abstand zwischen zwei aufeinanderfolgenden Wechselwirkungszentren geringer ist, als die Ausdehnung der Wechselwirkungszonen bzw. die Fokusspotgröße und dass die Fluence F unter dem zur Erzeugung optischer Durchbrüche erforderlichen Schwellwert M liegt.

Praktisch hat sich ein Abstand der Laserfoki bzw. Wechselwirkungszentren von ca. 3 - 5 μm als gut geeignet erwiesen, um mit möglichst geringer Pulsenergie und begrenztem Zeitaufwand Schnitte hoher Qualität zu erzeugen.

Bei einem laserchirurgischen Instrument 1, das sehr feine Schnitte erzeugt, beispielsweise wenn die eben geschilderten niedrigen Fluence-Werte für die Laserpulse verwendet werden, ist der Schnitt auch direkt nach der Erzeugung nicht sichtbar, entweder, weil Plasma- bzw. Gasblasen auftreten, die kleiner und kurzlebiger sind, als beim Betrieb außerhalb des Bereichs 18, oder weil überhaupt keine Blasen entstehen (bei Betrieb im Bereich 19). Die Präparation des isolierten Schnittes, beispielsweise mittels eines Spatels kann dann erschwert sein. Eine für viele Anwendungen verwendete manuelle Prozedur, bei der mit einem Spatel bzw. anderen Werkzeugen Restbrücken, die in der Schnittfläche noch nicht vollständig getrennt wurden, durchstoßen werden, kann bei einem glatten Schnitt sehr schwierig werden.

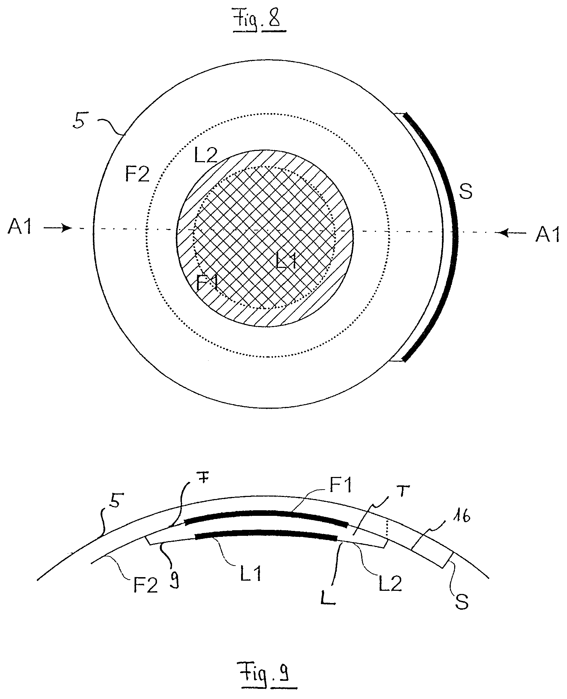

Um dies zu vermeiden, führt das Steuergerät 17 des laserchirurgischen Instrumentes 1 beispielsweise die in den Fig. 8 und 9 dargestellte Schnittzerlegung aus. Die Schnittfläche wird in Teilschnittflächen unterschiedlicher Feinheit zerlegt. Diese Teilschnittflächen werden unterschiedlich glatt geschnitten, so dass Bereiche bestehen, in denen die Schnittfläche optisch besser sichtbar ist, als in anderen.

Fig. 8 zeigt eine Draufsicht auf die Augenhornhaut 5 des Patientenauges 1 und Fig. 9 eine Schnittdarstellung entlang der Linie A1-A1 der Fig. 8. Wie zu sehen ist, ist die Schnittfläche 9 so gelegt, dass sie das Teilvolumen T isoliert, wie schon in Fig. 3 schematisch angedeutet war. Die Schnittfläche 9 besteht nun aus einem anterioren Abschnitt F und einem posterioren Abschnitt L. Der anteriore Abschnitt F wird über einen seitlich öffnenden Schnitt 16 zu einer Randöffnung S geführt, der zur Hornhautoberfläche führt. Das linsenförmige Teilvolumen T liegt also nach Ausführung der Schnittfläche 9 mit den Abschnitten F, L, 16 und S in einer durch die Randöffnung S gebildeten Tasche.

Damit ein Operateur diese Tasche mit einem Spatel oder einem anderen chirurgischen Instrument abfühlen kann, um eventuelle Gewebsbrücken zwischen dem linsenförmigen Teilvolumen T und dem Rest der Hornhaut 5 zu durchtrennen, ist der anteriore Abschnitt F wie auch der posteriore Abschnitt L jeweils in zwei Teilbereiche zerlegt. Ein Kernbereich F1 bzw. L1, der im Wesentlichen kreisförmig ist, wird jeweils von einem ringförmigen Randbereich F2 bzw. L2 umgeben. Im Kernbereich, der nahe der optischen Sehachse liegt, wird mit geringer Plasmablasengröße, d. h. mit einer feinen Schnittführung gearbeitet. Dies kann beispielsweise durch Betrieb in den Bereichen 18 bzw. 19 der Fig. 6 und 7 erfolgen. In den (ringförmigen) Randbereichen L2 und F2 wird dagegen ein vergleichsweise gröberer Schnitt erzeugt, beispielsweise indem bewusst außerhalb der Bereiche 18 oder 19 gearbeitet wird, so dass relativ große Plasmablasen entstehen. Die Schnittfläche ist somit in diesen Randbereichen sehr viel rauher und für den Operateur einfach zu erkennen.

Vorzugsweise sind die Durchmesser der zentralen Bereiche F1 und L1 größer als der Pupillendurchmesser P des behandelten Auges. Dadurch liegen die mit einer rauheren Schnittführung bearbeiteten Randbereiche F2 und L1 außerhalb des für die optische Wahrnehmung benutzten Bereiches der Hornhaut 5 und wirken sich nicht störend aus. Der Zweck der Zerlegung der Abschnitte L und F besteht darin, durch unterschiedliche Bearbeitung zugleich den Aspekt maximaler Schnittgenauigkeit als auch guter Handhabbarkeit durch Sichtbarkeit des Schnitte im Randbereich zu erreichen.

Arbeitet man zur Materialtrennung mit Plasmablasen, liegt die Energie der Laserpulse oberhalb des Schwellwertes M. Die Gestalt der Blasen, die das Ergebnis der Absorption der Laserenergie im Gewebe sind, unterliegt einer zeitlichen Änderung, wie bereits erwähnt. An eine erste Phase der Einzelblasenentstehung schließt sich eine Phase der Blasenagglomeration zusammen, in der sich mehrere Einzelblasen zu größeren Makroblasen zusammenschließen. Als letzte Phase ist schließlich die Dissipation zu verzeichnen, bei welcher der Gasgehalt der Makroblasen vom umliegenden Gewebe aufgenommen wird, bis die Blasen schließlich wieder vollends verschwunden sind. Makroblasen haben nun die störende Eigenschaft, das umliegende Gewebe zu deformieren. Wird in das deformierte Gewebe an eine bestimmte Position ein weiteres Wechselwirkungszentrum als Beginn einer Plasmablase gelegt, so verändert sich die Lage des Wechselwirkungszentrums und mithin die Lage der dadurch bewirkten Gewebetrennung, sobald die Dissipationsphase einsetzt, in der die Blasen verschwinden und sich das deformierte Gewebe (zumindest teilweise) relaxiert. Da sich die Makroblasen erst nach einer charakteristischen Zeit bilden und nicht schon direkt nach Einbringung der Laserpulsenergie vorhanden sind, ist für eine Variante des laserchirurgischen Instrumentes 1 vorgesehen, dass die Zeit zwischen der Applikation der Laserenergie in zwei sich potentiell beeinflussenden Gebieten des Gewebes so gering gehalten wird, dass sie kleiner ist, als eine charakteristische Zeitdauer, welche für die Bildung von Makroblasen erforderlich ist.

Bei der Isolierung des linsenförmigen Teilvolumens T sind einander störend beeinflussende Gebiete der posteriore und der anteriore Abschnitte der Schnittfläche 9 im Bereich der optischen Sehachse. Erzeugt man den Schnitt im anterioren Abschnitt F der Schnittfläche 9 erst zu einem Zeitpunkt, zu dem der zuvor bearbeitete posteriore Abschnitt L bereits Makroblasen aufweist, liegt die Schnittfläche des anterioren Abschnittes F in deformiertem Gewebe. Das Ergebnis wäre nach Relaxation des Gewebes eine unerwünschte Welligkeit der Schnittfläche 9 im anterioren Abschnitt F. Das laserchirurgische Instrument 1 erzeugt deshalb die Schnittfläche im anterioren Abschnitt F und im posterioren Abschnitt L in einem zeitlichen Abstand, der geringer ist, als die charakteristische Zeitdauer, in der Makroblasen entstehen. Diese Zeitdauer liegt typisch bei etwa 5 s.

Eine Möglichkeit dies zu bewerkstelligen liegt darin, den anterioren sowie den posterioren Abschnitt in entsprechende Teilflächen aufzuteilen und bei der Schnittflächenerzeugung zwischen den Teilflächen des posterioren bzw. anterioren Abschnittes zu wechseln, so dass zumindest im zentralen Bereich die charakteristische Zeitdauer bei der Erzeugung der Teilflächen, posterior und anterior, nicht überschritten wird. Eine andere Möglichkeit liegt in einer geeigneten Aneinanderreihung der Wechselwirkungszentren. So kann beispielsweise zuerst der posteriore Abschnitt L mit einer von außen nach innen zur optischen Sehachse führenden Spirale und direkt anschließend der anteriore Abschnitt F mit einer von der Sehachse nach außen laufenden Spirale geschnitten werden. Dann liegen zumindest in einem Kernbereich rund um die Sehachse die erzeugten Wechselwirkungen innerhalb des durch die charakteristische Zeitdauer vorgegebenen Zeitfensters, so dass keine Makroblasen-Beeinflussung bei der Bearbeitung des anterioren Abschnittes gegeben ist.

Bei der Aufteilung der Teilflächen, die das laserchirurgische Instrument 1 gesteuert durch die Steuereinrichtung 17 vornimmt, ist dafür Sorge getroffen, dass ein zu bearbeitender posteriorer Bereich nicht von einer als Streuzentrum wirkenden, bereits bearbeiteten anterioren Fläche oder Wechselwirkungszone gestört wird.

Die geschilderten Schnittformen, Flächenaufteilungen, etc. werden vom laserchirurgischen Instrument unter Steuerung der Steuereinrichtung 17 vorgenommen. Die Steuereinrichtung 17 bewirkt den Betrieb des laserchirurgischen Instrumentes 1 mit den hier beschriebenen Verfahrensmerkmalen.

Soweit vorangehend Ausgestaltungen des laserchirurgischen Instrumentes geschildert sind, können diese allein wie auch in Kombination realisiert sein, je nach konkreter Realisierung des laserchirurgischen Instrumentes 1. Auch kann das Instrument 1 anstelle eines Einsatzes in der Laserchirurgie auch für die nicht chirurgische Materialbearbeitung verwendet werden, beispielsweise bei der Erzeugung von Wellenleitern oder der Bearbeitung flexibler Materialien.

**Patentansprüche**

**1.** Vorrichtung zur Bearbeitung von Material (5) eines Auges (1) mittels Laserstrahlung, mit einer Laserstrahlquelle (S), die zur Abgabe gepulster Bearbeitungs-Laserstrahlung (3) zur Wechselwirkung mit dem Material (5) eingerichtet ist, einer die gepulste Bearbeitungs-Laserstrahlung (3) entlang einer optischen Achse (H) in das Material (5) auf ein Wechselwirkungszentrum (7) fokussierenden Optik (6), einer die Lage des Wechselwirkungszentrums im Material (5) verstellenden Scaneinrichtung (10), wobei jeder Bearbeitungs-Laserpuls in einer das ihm zugeordnete Wechselwirkungszentrum (7) umgebenden Zone (8) mit dem Material (5) wechselwirkt, so dass in den Wechselwirkungszonen (8) Materials (5) trennbar ist, und einer Steuereinrichtung (17) zur Ansteuerung der Scaneinrichtung (10) und der Laserstrahlquelle (S) derart, dass im Material (5) durch Aneinanderreihen von Wechselwirkungszonen (8) eine Schnittfläche (9) entsteht, wobei durch die Steuereinrichtung (17) die Laserstrahlquelle (S) und die Scaneinrichtung (10) so ansteuerbar sind, dass die Schnittfläche (9) zwei entlang der optischen Achse (H) benachbarte Abschnitte (F, L) und zwar einen anterioren und einen posterioren Abschnitt, die jeweils in eine zentrale kreisförmige und mindestens eine umgebende ringförmige Teilfläche unterteilt sind, aufweist wobei die zentralen Teilflächen in einem zeitlichen Abstand $t \leq 5\,s$ mit gepulster Bearbeitungs-Laserstrahlung (3) beaufschlagbar sind, so dass in den zentralen Teilflächen der benachbarten Abschnitte (F, L) die Schnittfläche innerhalb von maximal 5 s bildbar ist, wobei in der jeweiligen Teilfläche die Schnittfläche zuerst im posterioren und dann im anterioren Abschnitt erzeugbar ist.

2. Vorrichtung nach Anspruch 1, **dadurch gekennzeichnet, dass** die zwei der entlang der optischen Achse (H) benachbarten Abschnitte (F, L) einen anterioren Abschnitt (F) und einen posterioren Abschnitt (L) umfassen und durch die Steuereinrichtung (17) die Laserstrahlquelle (S) und die Scaneinrichtung (10) so ansteuerbar sind, dass der posteriore Abschnitt (L) mit einer von außen nach innen verlaufenden Spirale der Wechselwirkungszentren und der anterioren Abschnitt (F) mit einer von innen nach außen verlaufenden Spirale erzeugbar sind.

3. Vorrichtung nach Anspruch 1 oder 2, **dadurch gekennzeichnet, dass** durch die Steuereinrichtung (17) die entlang der optischen Achse (H) benachbarten Abschnitte (L, F) jeweils in Teilschnittflächen (F1, F2; L1, L2) aufteilbar sind und die Laserstrahlquelle (S) und die Scaneinrichtung (10) so ansteuerbar sind, dass entlang der optischen Achse (H) benachbarte Teilschnittflächen (F1, F2; L1, L2) unmittelbar zeitlich aufeinanderfolgend durch das Aneinanderreihen der Wechselwirkungszentren (7) mit Bearbeitungs-Lasterstrahlung (3) beaufschlagbar sind.

4. Vorrichtung nach einem der obigen Ansprüche, **dadurch gekennzeichnet, dass** durch die Steuereinrichtung (17) die Laserstrahlquelle (S) und die Scaneinrichtung (10) so ansteuerbar sind, dass benachbarte Wechselwirkungszentren (7) in einem örtlichen Abstand $a \leq 10\,\mu m$ zueinander liegen.

5. Vorrichtung nach der obigen Ansprüche, **dadurch gekennzeichnet, dass** für jedes Wechselwirkungszentrum (7) eine Bearbeitungs-Laserpuls-Fluence F unter 5 J/cm$^2$ liegt.

6. Vorrichtung nach Anspruch 5, **dadurch gekennzeichnet, dass** die Fluence jedes Bearbeitungs-Laserpulses über einem Schwellwert M liegt, der durch

$$M = 3{,}3\ \text{J/cm}^2 - (2{,}4\ \text{J/cm}^2) / (1 + (a/r)^2)$$

gegeben ist, wobei a der Abstand zwischen zwei benachbarten Wechselwirkungszentren (7) und r ein Parameter mit $3\,\mu m \leq r \leq 10\,\mu m$ ist, wobei bevorzugt die Fluence F jedes Bearbeitungs-Laserpulses um maximal 3 J/cm$^2$ über dem Schwellwert M liegt.

7. Vorrichtung nach einem der obigen Ansprüche, **dadurch gekennzeichnet, dass** die Laserstrahlquelle ein Laser einer Gefährdungsklasse unter 3, vorzugsweise ein Laser der Gefährdungsklasse 1M, ist.

8. Vorrichtung nach einem der obigen Ansprüche, **dadurch gekennzeichnet, dass** die zentralen Teilflächen einen Durchmesser größer als einen Pupillendurchmesser (P) des Auges (1) aufweisen.

**Claims**

1. A device for processing material (5) of an eye (1) by means of laser radiation, said device comprising a source of laser radiation (S) adapted to emit pulsed processing laser radiation (3) for interaction with the material (5), optics (6) focusing the pulsed processing laser radiation (3) along an optical axis (H) to a center of interaction (7) in the material (5), a scanning unit (10) shifting the position of the center of interaction within the material (5), wherein each processing laser pulse interacts with the material (5) in a zone (8) surrounding the center of interaction (7) assigned to said laser pulse so that material (5) is separable in the zones of interaction (8), and a control unit (17) for controlling the scanning unit (10) and the source of laser radiation (S) so that a cut surface (9) is produced in the material (5) by sequential arrangement of zones of interaction (8), wherein the source of laser radiation (S) and the scanning unit (10) are controllable by the control unit (17) such that the cut surface (9) comprises two portions (F, L), namely an anterior and a posterior portion, adjacent along an optical axis (H) which portions are each subdivided into one round central surface part and at least one surrounding ring-shaped surface part, wherein pulsed processing laser radiation (3) is applicable to the central surfaces parts within a time interval of $t \leq 5$ s so that the cut surface is producible in the central surface parts of the adjacent portions (F, L) within 5 s at most, wherein the cut surface in the respective surface part is producible first in the posterior portion, followed by the anterior portion.

2. The device of claim 1, **characterized in that** the two adjacent portions (F, L) arranged along the optical axis (H) comprise an anterior portion (F) and a posterior portion (L), and the source of laser radiation (S) and the scanning unit (10) are controllable by the control unit (17) such that the posterior portion (L) is producible by a spiral of centers of interactions stretching from outside to inside and the anterior portion (F) by a spiral stretching from inside to outside.

3. The device of claim 1 or 2, **characterized in that** the adjacent portions (L, F) arranged along the optical axis (H) are separable by the control unit (17) into respective surface parts (F1, F2; L1, L2) and the laser radiation source (S) and the scanning unit (10) are controllable such that processing laser radiation (3) is applicable to surface parts (F1, F2; L1, L2) adjacent along the optical axis (H) in immediate temporal sequence by sequential arrangement of the centers of interaction (7).

4. The device as claimed in any of the above claims, **characterized in that** the laser radiation source (S) and the scanning unit (10) are controllable by the control unit (17) such that adjacent centers of interaction (7) are located in a spatial distance $a \leq 10$ μm.

5. The device as claimed in any of the above claims, **characterized in that** a fluence F of processing laser radiation pulses is below 5 J/cm$^3$ for each center of interaction (7).

6. The device as claimed in claim 5, **characterized in that** the fluence of each processing laser radiation pulse is below a threshold value M given as

$$M = 3.3 \text{ J/cm}^2 - (2.4 \text{ J/cm}^2) / (1 + (a/r)^2),$$

wherein a is the distance between two adjacent centers of interaction (7) and r is a parameter, with $3 \text{ μm} \leq r \leq 10 \text{ μm}$, wherein, preferably, the fluence F of each processing laser radiation pulse exceeds the threshold M by 3 J/cm$^3$ at most.

7. The device as claimed in any of the above claims, **characterized in that** the laser radiation source is a laser of a hazard class below 3, preferably a laser of hazard class 1 M.

8. The device as claimed in any of the above claims, **characterized in that** the central surface parts comprise a diameter larger than the pupil diameter (P) of an eye (1).

**Revendications**

1. Dispositif de traitement de la matière (5) d'un oeil (1) au moyen d'un rayonnement laser, comprenant une source de rayon laser (S) qui est conçue pour délivrer un rayonnement laser de traitement pulsé (3) destiné à interagir avec la matière (5), une optique (6) qui concentre le rayonnement laser de traitement pulsé (3) le long d'un axe optique (H) dans la matière (5) sur un centre d'interaction (7), un appareil de balayage (10) qui ajuste la position du centre d'interaction dans la matière (5), chaque impulsion laser de traitement interagissant avec la matière (5) dans une zone (8) qui entoure le centre d'interaction (7) qui lui est associé, de sorte que de la matière (5) peut être enlevée dans les zones d'interaction (8), et un appareil de commande (17) destiné à commander l'appareil de balayage (10) et la source de rayon laser (S) de telle sorte qu'il se produit une surface de coupe (9) dans la matière (5) par juxtaposition des zones d'interaction (8), la source de rayon laser (S) et l'appareil de balayage (10) pouvant être commandés par l'appareil de commande (17) de telle sorte que la surface de coupe (9) possède deux portions (F, L) voisines le long de l'axe optique (H), à savoir une portion antérieure et une postérieure, lesquelles sont respectivement subdivisées en une surface partielle centrale de forme circulaire et au moins une surface partielle périphérique de forme annulaire, les surfaces partielles centrales pouvant être soumises au rayonnement laser de traitement pulsé (3) à un intervalle de temps t ≤ 5 s, de sorte que la surface de coupe peut être formée en un maximum de 5 s dans les surfaces partielles centrales des portions (F, L) voisines, la surface de coupe dans la surface partielle correspondante pouvant d'abord être produite dans la portion postérieure et ensuite dans la portion antérieure.

2. Dispositif selon la revendication 1, **caractérisé en ce que** les deux portions (F, L) voisines le long de l'axe optique (H) comprennent une portion antérieure (F) et une portion postérieure (L) et la source de rayon laser (S) et l'appareil de balayage (10) peuvent être commandés par l'appareil de commande (17) de telle sorte que la portion postérieure (L) peut être produite avec une spirale des centres d'interaction s'étendant de l'extérieur vers l'intérieur et la portion antérieure (F) avec une spirale qui s'étend de l'intérieur vers l'extérieur.

3. Dispositif selon la revendication 1 ou 2, **caractérisé en ce que** les portions (L, F) voisines le long de l'axe optique (H) peuvent respectivement être partagées en surfaces de coupe partielles (F1, F2 ; L1, L2) par l'appareil de commande (17) et la source de rayon laser (S) et l'appareil de balayage (10) peuvent être commandés de telle sorte que les surfaces de coupe partielles (F1, F2 ; L1, L2) voisines le long de l'axe optique (H) peuvent être soumises au rayonnement laser de traitement (3) directement successivement dans le temps par la juxtaposition des centres d'interaction (7).

4. Dispositif selon l'une des revendications précédentes, **caractérisé en ce que** la source de rayon laser (S) et l'appareil de balayage (10) peuvent être commandés par l'appareil de commande (17) de telle sorte que les centres d'interaction (7) voisins se trouvent à un écart local a ≤ 10 μm les uns des autres.

5. Dispositif selon l'une des revendications précédentes, **caractérisé en ce que** pour chaque centre d'interaction (7), une fluence F d'impulsion laser de traitement est inférieure à 5 J/cm$^2$.

6. Dispositif selon la revendication 5, **caractérisé en ce que** la fluence de chaque impulsion laser de traitement est supérieure à une valeur de seuil M, laquelle est donnée par

$$M = 3,3 \ \text{J/cm}^2 - (2,4 \ \text{J/cm}^2) \ / \ (1+(a/r)^2),$$

où a désigne l'écart entre deux centres d'interaction (7) voisins et r désigne un paramètre tel que 3 μm ≤ r ≤ 10 μm, la fluence F de chaque impulsion laser de traitement étant de préférence supérieure au maximum de 3 J/cm$^2$ à la valeur de seuil M.

7. Dispositif selon l'une des revendications précédentes, **caractérisé en ce que** la source de rayon laser est un laser d'une classe de risque inférieure à 3, de préférence un laser de la classe de risque 1M.

8. Dispositif selon l'une des revendications précédentes, **caractérisé en ce que** les surfaces partielles centrales possèdent un diamètre supérieur à un diamètre de la pupille (P) de l'oeil (1).

FIG 1

FIG 2

## FIG 3

## FIG 4

## FIG 5

18.1

$\overline{F}$max

18

18.2

$a$max

M

F [ J/cm² ]

0    1  2  3  4  5  6  7  8  9  10 11 12 13 14 15 16 17 18 19 20 21 22 23 24

$a$ [ μm ]

Fig. 6

19

M

F [ J/cm² ]

0    1  2  3  4  5  6  7  8  9  10 11 12 13 14 15 16 17 18 19 20 21 22 23 24

$a$ [ μm ]

d

Fig. 7

Fig. 8

Fig. 9

Fig. 12

Fig. 11

Fig. 10

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- DE 10202036 A1
- WO 2005011546 A1
- US 2005165387 A1
- US 2003212387 A1
- US 5984916 A

### In der Beschreibung aufgeführte Nicht-Patentliteratur

- **A. HEISTERKAMP et al.** *Der Ophthalmologe,* 2001, vol. 98, 623-628